# EUROPEAN PATENT APPLICATION

(11) **EP 4 495 260 A1**
(43) Date of publication of application: **22.01.2025**
(21) Application number: 23186687.2
(22) Date of filing: 20.07.2023
(51) Int. Cl.: C12Q 1/6883

(54) **METHOD FOR DIAGNOSING ANEURYSM**

(71) Applicant: Twobull Meditherapy P.C., 26222 Patra (GR)
(72) Inventor: TOUMPOULIS, Ioannis, 25200 KATO ACHAIA (GR)
(74) Representative: ZBM Patents - Zea, Barlocci & Markvardsen

(57) **Abstract**

The present disclosure refers to an *in vitro* method for diagnosing an aneurysm in a subject, the method comprising determining in an isolated biological sample from the subject the level of one or more microRNAs (miRNA) selected from the group consisting of hsa-miR-223-3p, hsa-miR-199a-3p and hsa-miR-335-5p, wherein, when the level of the one or more miRNAs is lower than a corresponding reference value, this is indicative that the subject suffers from an aneurysm.

## Description

### Technical Field

The present invention pertains to the field of diagnosis, in particular diagnosis of aneurysm by detecting biomarkers in blood samples.

### Background Art

An aneurysm is an outward bulging, likened to a bubble or balloon, caused by a localized, abnormal, weak spot on a blood vessel wall. Although aneurysms may occur in any blood vessel, particularly dangerous examples include aneurysms of the Circle of Willis in the brain, and aortic aneurysms affecting the abdominal aorta (abdominal aortic aneurysms), the thoracic aorta (thoracic aortic aneurysms), or both the thoracic and the abdominal aorta (thoracoabdominal aortic aneurysms).

An aneurysm usually progresses asymptomatically until rupture. The rupture of an aneurysm, which is usually the first and, simultaneously, the last symptom, leads to uncontrolled internal bleeding which often leads to death. Rupture of an aneurysm carries a mortality rate of 75% or higher, being a considerable leading cause of death worldwide. Moreover, it is estimated that the incidence of aortic aneurysms will continue to increase worldwide in the next years because of the aging of the general population. The pathogenesis and the molecular mechanisms which are controlling the aneurysm formation and progression are under investigation.

Due to their relative lack of clinical symptoms, aneurysms are often discovered incidentally during a routine check-up or other screening. Imaging techniques such as using magnetic resonance imaging (MRI), computerised tomography (CT), and ultrasound imaging can detect and assist in aneurysm diagnosis. However, these imaging techniques are expensive, available only in hospitals, and not available for screening, even if only in population at risk of suffering from aneurysms. At present, there is no simple laboratory test that has the ability to reliably detect aneurysms.

EP3794148 recently disclosed a method for diagnosing aneurysm by determining a group of biomarkers in blood samples. While representing a great step forward in the field, there is ample room for improvement in terms of sensitivity and reproducibility.

In view of the above, there is still the need to provide improved diagnostic tests for diagnosing aneurysm, particularly in the early stages, in order to apply appropriate preventive and/or corrective measures as well as to provide a prognostic algorithm in facilitating clinical decision making.

### Summary of Invention

The inventor has surprisingly found that a more accurate and reliable diagnosis of aneurysm may be obtained by determining specific microRNAs (miRNAs).

Thus, in a first aspect, the present disclosure provides an *in vitro* method for diagnosing an aneurysm in a subject, the method comprising determining in an isolated biological sample from the subject the level of one or more miRNAs selected from the group consisting of hsa-miR-223-3p, hsa-miR-199a-3p and hsa-miR-335-5p.

These miRNAs (hsa-miR-223-3p, hsa-miR-199a-3p and hsa-miR-335-5p) were found to be significantly downregulated in plasma samples of patients with aneurysm, compared to healthy controls. Accordingly, when the level of these miRNAs is lower than a corresponding reference value, this is indicative that the subject suffers from an aneurysm. Moreover, as shown in the examples below, the predictive value to discriminate aneurysm patients from controls was evaluated by receiver operating characteristic (ROC) curve analysis and it was found that all three miRNAs (hsa-miR-223-3p, hsa-miR-199a-3p and hsa-miR-335-5p) are able to independently diagnose the presence of an aneurysm with sensitivity above 90% and specificity above 85%.These results support the discovery that the present method may accurately and rapidly discriminate aneurysm patients from controls with high specificity and sensitivity. Moreover, these high predictive values were obtained by determining the differential expression of miRNAs in peripheral blood samples. The present method thus, allows for early diagnosis of aneurysm, without the need of expensive imaging techniques or invasive procedures, using a simple blood sample from the patient.

It is also important that the present diagnostic method is highly reproducible. Unlike methods that rely on determining the level of gene expression through mRNA quantification, the present method is based on determining microRNA(s) expression levels, which is a remarkably stable molecule compared to mRNA and, consequently, more reliably detectable and quantifiable in biofluids.

It is also remarkable that the diagnosis of aneurysm may be performed by determining a reduced number of biomarker miRNAs. Indeed, any one of hsa-miR-223-3p, hsa-miR-199a-3p or hsa-miR-335-5p can independently provide a good diagnosis by themselves. Moreover, combination of these biomarkers provides an even more accurate diagnosis with increased sensitivity and specificity.

Another advantage is that the present method allows the diagnosis of different types of major aneurysms, such as thoracic aortic aneurysm, abdominal aortic aneurysm, and intracranial aneurysm. Some aneurysms are difficult to diagnose by prior art methods, which further highlights the relevant contribution to the art of the present method.

Aneurysms, in particular large diameter aneurysms, are considered at risk of rupture. Rupture of the vessel, such as the aorta, results in massive internal bleeding and, unless treated immediately, shock and death can occur quickly. Surgery is recommended to avoid the rupture if the size of the aneurysm is reaching specific diameters (e.g. above 5 cm in diameter in the abdominal aorta and in the ascending thoracic aorta or above 6 cm in the descending thoracic aorta) and/or it is growing rapidly (e.g. more than 0.5 cm per year). Up to now, the most cost-efficient screening test, to determine if a patient has an aneurysm at risk for rupture, is performed by CT scan study with contrast. The inventor has found that determining the level of microRNAs described herein, in particular, hsa-miR-223-3p, hsa-miR-199a-3p and/or hsa-miR-335-5p can accurately predict the risk of rupture of an aneurysm. As shown in the examples below a correlation has been found between the size of the aneurysm and the level of expression of the micorRNAs.

Accordingly, a second aspect of the present disclosure refers to an *in vitro* method for the prognosis of an aneurysm in a subject, the method comprising determining in an isolated biological sample from the subject the level of one or more miRNAs selected from the group consisting of hsa-miR-223-3p, hsa-miR-199a-3p, and hsa-miR-335-5p. In particular, the prognosis refers to the risk of rupture of the aneurysms and, therefore, the disclosure also provides an *in vitro* method for determining the risk of rupture of an aneurysm. When the level of expression determined for each one of the one or more miRNAs is ≥80% decreased (0.20-fold change) with respect to a corresponding reference value this is indicative that the subject suffers from risk of rupture of an aneurysm, which is a bad prognosis.

The present method constitutes a reliable, convenient and more cost-effective test for identifying patients with aneurysms at risk for rupture.

Appropriate medical intervention may be recommended by the clinician in view of the level of the miRNAs disclosed herein after a simple blood sampling. Additionally, the patient may be subjected to a tight follow-up schedule if so needed. Thus, in a third aspect, the disclosure also refers to a method of recommending a medical regimen for a subject suspicious of suffering from aneurysm or bad prognosis, e.g. is at risk of rupture of an aneurysm, the method comprising:
(a) diagnosing if the subject suffers from aneurysm or has a bad prognosis for an aneurysm, e.g. is at risk of rupture of an aneurysm, by the method as defined in the first or second aspects, and
(b) recommending an appropriate therapeutic regime for aneurysm if the subject is diagnosed of suffering from an aneurysm or an appropriate therapeutic regime for bad prognosis, e.g. risk of rupture of an aneurysm, if the subject is determined to have a bad prognosis, e.g. a risk of suffering from rupture of an aneurysm.

Additionally, by determining the level of the herein disclosed miRNAs, patients having an aneurysm who have received a specific treatment may be monitored in order to ensure the effectiveness of the therapeutic intervention (i.e. insertion of endovascular prosthesis) or to warn early of the risk for rupture of the affected blood vessel. The present method allows for easy follow-up and improved management of patients with aneurysms, thus avoiding unnecessary suffering and/or minimizing side effects of radiation and injection of contrast affecting renal function.

A fourth aspect thus refers to a method for determining the response of a patient that suffers from an aneurysm to medical regime for aneurysm, the method comprising:
(a) determining in an isolated biological sample from the subject the level of one or more miRNAs selected from the group consisting of hsa-miR-223-3p, hsa-miR-199a-3p and hsa-miR-335-5p, and comparing said level with the level of the same miRNA determined for the same patient before the start of the therapy or at an earlier phase of the therapy, wherein a decrease in the level of the miRNA with respect to start the therapy or earlier phase of the therapy is indicative of a bad response.

The fourth aspect can also be seen as an *in vitro* method for recommending an alternative and/or complementary therapeutic regime for a patient having an aneurysm, the method comprising the steps (a) and (b) as defined above, wherein a decrease in the level of the miRNA with respect to start of the therapy or earlier phase of the therapy indicates that an alternative and/or complementary therapeutic regime is needed. This may also be formulated as a method for treating a patient having an aneurysm that is not responding to a therapeutic regime for aneurysm, said method comprising the steps (a) and (b) as defined above, and administering alternative and/or complementary therapeutic regime for aneurysm when there is a decrease in the level of the miRNA with respect to start the therapy or earlier phase of the therapy. Sometimes the clinician may even recommend or administer an alternative and/or complementary therapy when the level of the miRNA is unchanged with respect to start of the therapy or earlier phase of the therapy.

The present method may also provide early information on the risk of relapses in patients that have been treated for aneurysm (i.e. open surgery or insertion of endovascular prosthesis). A successful therapeutic intervention will result in levels of the miRNA that are elevated and/or closer to controls. However, one patient who has developed an aneurysm remains at risk to develop another aneurysm at another site. Early detection and subsequent management of relapse or detection of new onset aneurysm at another site may highly improve the prognosis of the patient suffering from aneurysm(s).

Thus, a fifth aspect refers to an *in vitro* method for detecting relapse in a subject that has been treated for aneurysm or development of a new onset aneurysm at another site, the method comprising determining in an isolated sample from the subject the level of one or more miRNA selected from the group consisting of hsa-miR-223-3p, hsa-miR-199a-3p and hsa-miR-335-5p, wherein when the level of each one of the one or more miRNAs is lower than a corresponding reference value this is indicative that the subject is in high risk of suffering a relapse or development of new onset aneurysm at another side of the arterial tree.

A sixth aspect refers to use of a combination of miRNAs comprising at least hsa-miR-223-3p, hsa-miR-199a-3p and hsa-miR-335-5p as biomarkers for diagnosing aneurysm or, alternatively, for the prognosis of an aneurysm, including determining the risk of rupture of an aneurysm or, alternatively, for detecting relapse in a subject that has been treated for aneurysm.

A seventh aspect refers to use of means for determining the level of one or more microRNAs selected from the group consisting of hsa-miR-223-3p, hsa-miR-199a-3p, and hsa-miR-335-5p, in any of the methods as defined in aspects 1-5. Said means may form part of a kit. Thus, an eight aspect refers to a kit comprising means for determining the level of hsa-miR-223-3p, hsa-miR-199a-3p, and hsa-miR-335-5p. A ninth aspect further provides the use of said kit in any of the methods as defined in aspects 1-5.

### Brief Description of Drawings

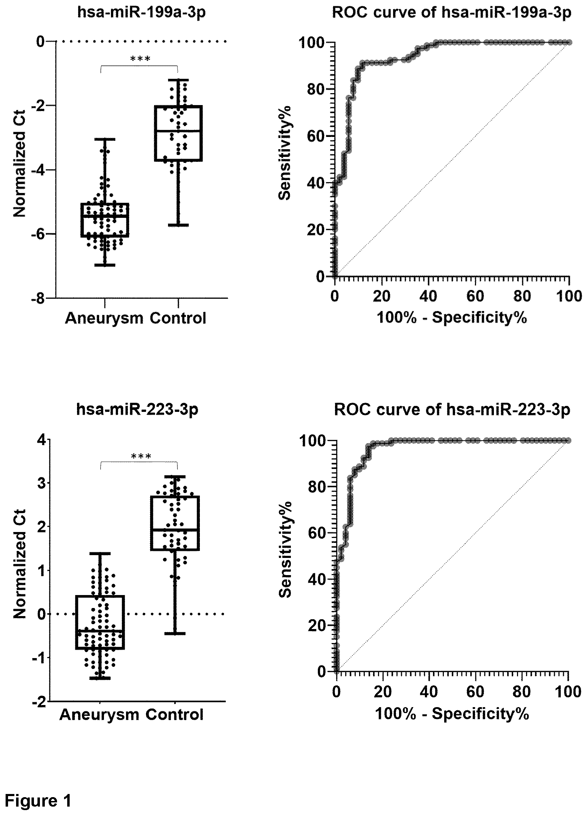

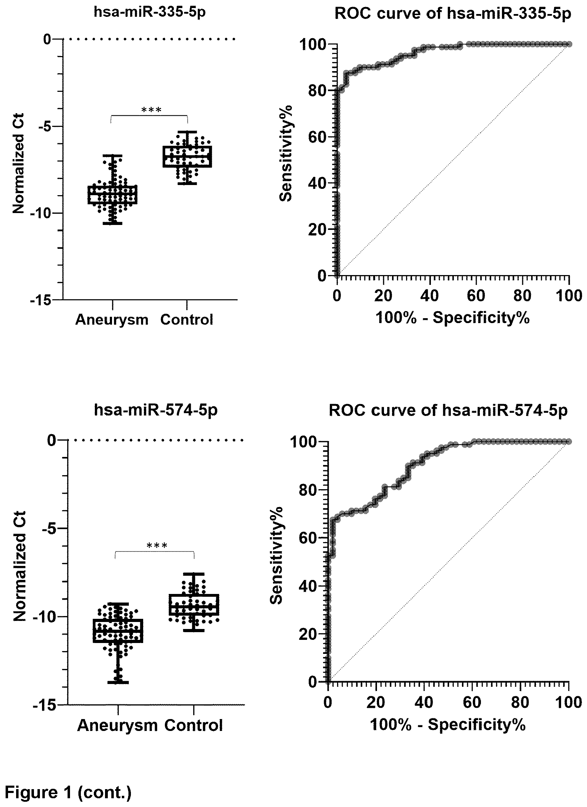

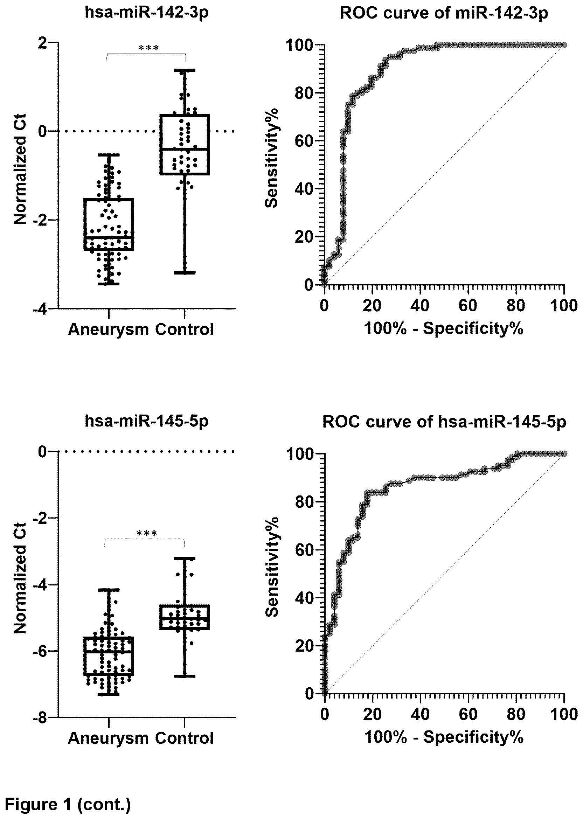

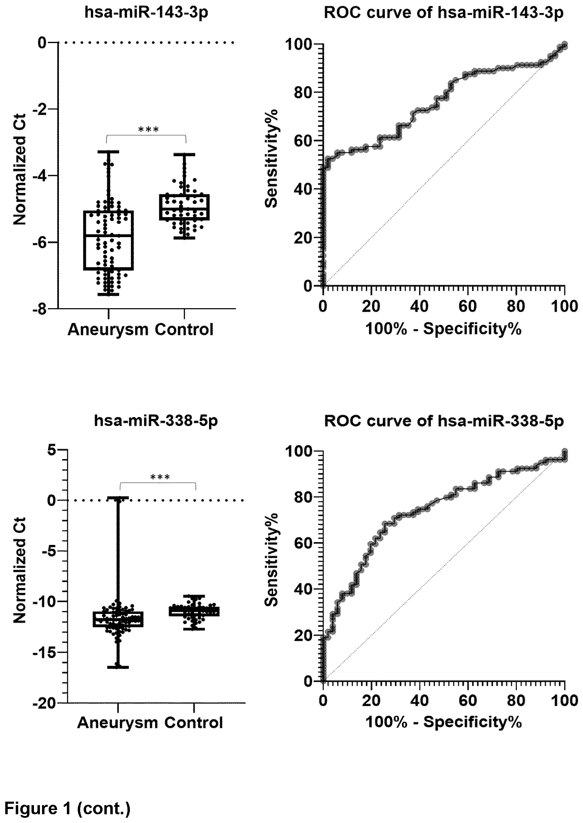

Figure 1: On the left, it is shown the differential expression levels of eight plasma miRNAs in patients with aneurysm versus healthy controls (subjects without aneurysm). miRNA expression data were normalized to UniSp4 levels. On the right, it is shown the receiver operating characteristic (ROC) curve analysis of each miRNA individually between aneurysm patients and healthy controls. ***p value<0.0001

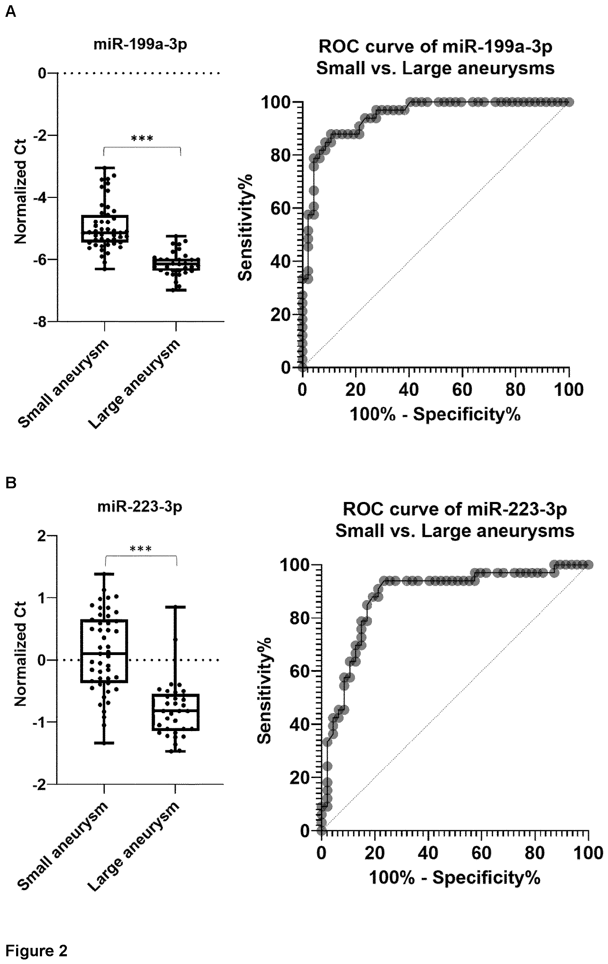

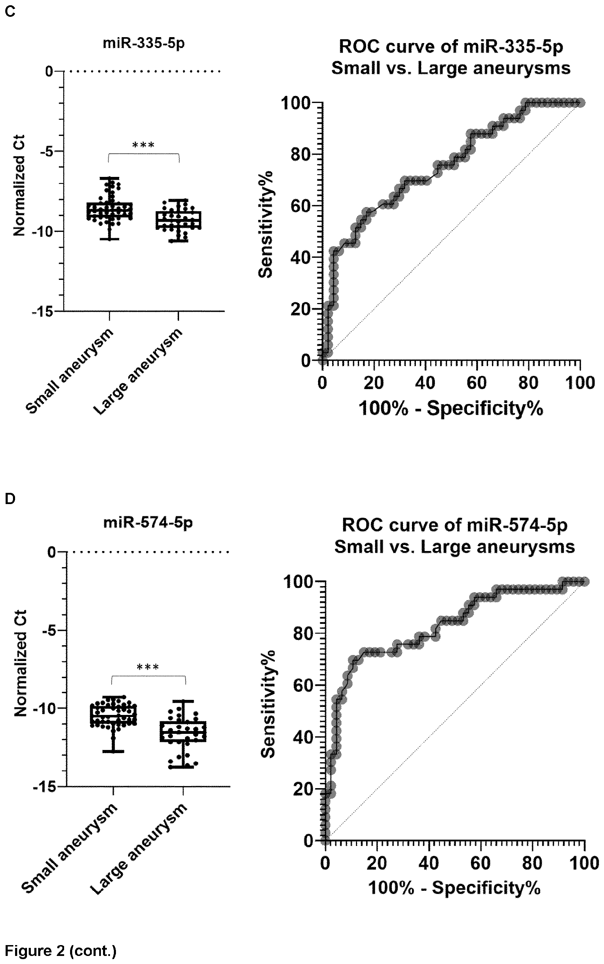

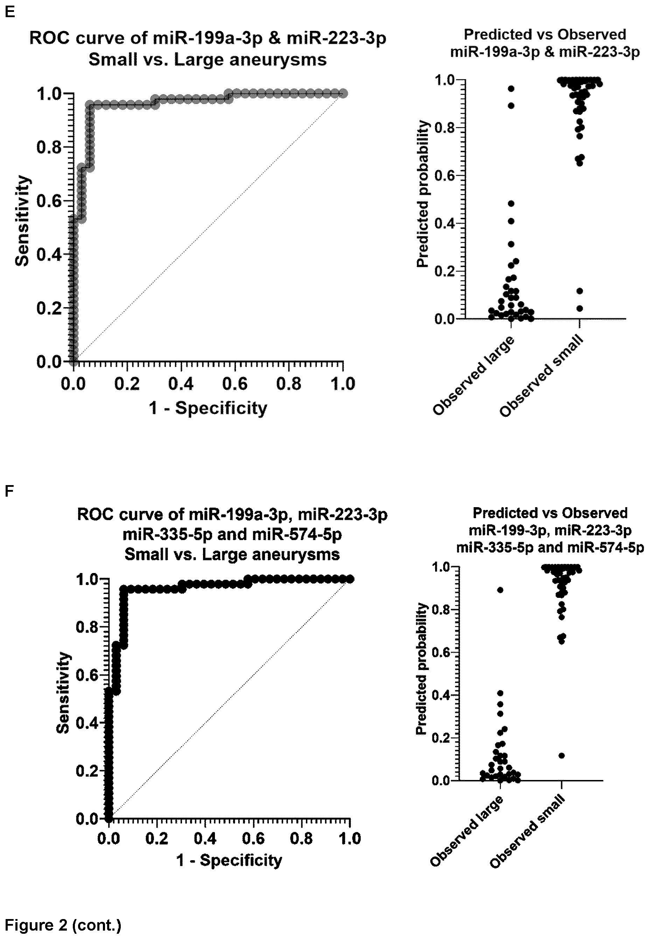

Figure 2: On the left, it is shown the differential expression levels of four miRNAs in patients with small diameter aneurysms versus large diameter aneurysms (panels A through D). miRNA expression data were normalized to UniSP4 levels. On the right, it is shown the ROC curve analysis of each miRNA individually between patients with small diameter aneurysms and patients with large diameter aneurysms. ***p value<0.001. Panels E and F depict the increased predictive value of two combined and all four combined miRNAs respectively.

### Detailed description of the invention

The present disclosure is related to a method for diagnosing aneurysm by determining the level of one or more of the herein disclosed microRNAs.

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

It must be noted that, as used in this specification and the intended claims, the singular forms "a," "an," and "the" include plural referents unless the context clearly dictates otherwise.

The term "diagnosis" is known to the person skilled in the art. As used herein "diagnosis" is understood as becoming aware of a particular medical condition, complication; the determination of the nature of the condition; or the distinguishing of the condition from another. It refers both to the process of attempting to determine or identify the possible condition, and to the opinion reached by this process. A diagnosis, in the sense of diagnostic procedure, can be regarded as an attempt at classification of an individual's condition into separate and distinct categories that allow medical decisions about treatment and prognosis to be made. Subsequently, a diagnostic opinion is often described in terms of a condition. "Prognosis" as used herein refers to the prediction of the probable progression and outcome of the disease as well as the monitoring of the disease progression. The prognosis may include, for example, determining the risk of rupture of an aneurysm. A patient which has been determined to be at risk of rupture of an aneurysm according to the present disclosure is a patient that has a bad prognosis.

MicroRNAs (miRNAs) are a large group of highly conserved, small noncoding RNAs (generally 21 to 25 nucleotides in length), which usually suppress gene expression at the post-transcriptional level. miRNAs are transcribed by RNA polymerase II either from independent genes or from introns of protein-coding genes (mirtrons). The transcribed primary molecules (pri-miRNAs) are processed by a member of the ribonuclease (RNase) III family, named Drosha, forming an approximately 70-nucleotide stem-loop precursor miRNA (pre-miRNA), which is exported to the cytoplasm, where Dicer- another RNase III enzyme - further cleaves it to an ~20-bp mature miRNA and antisense miRNA star (miRNA/miRNA*) duplex. Their mechanism of action involves the incorporation of mature miRNA into the miRNA-induced silencing complex (miRISC) and the subsequent, nearly perfect base-pairing with sequences in the 3' untranslated region (3' UTR) of target-mRNA, leading to translational repression and/or mRNA degradation.

The term "reference value" in the context of the present invention is to be understood as a predefined level of expression product of the genes in a sample or group of samples. This value is used as a threshold to discriminate subjects wherein the condition to be analysed is present from those wherein such condition is absent. The samples are taken from a well-defined control subject or group of control subjects not having the disease or any condition that is related with the disease. The skilled person in the art, making use of the general knowledge, is able to choose the subject or group of subjects more adequate for obtaining the reference value. Methods for obtaining the reference value from the group of subjects selected are well known in the state of the art. In one embodiment of the present invention, the reference value is determined from a subject or group of subjects that do not suffer from aneurysm. In a particular embodiment the reference value is determined from a healthy subject or group of healthy subjects.

In the sense of the present invention the terms "the level of microRNA" and "the level of expression of microRNA" are used interchangeably.

As outlined above, the inventor has found that some miRNAs are differentially expressed in patients suffering from aneurysm and provide an accurate diagnosis of this condition with high sensitivity and specificity. Said miRNAs are mainly: hsa-miR-199a-3p, hsa-miR-223-3p and hsa-miR-335-5p. However, other miRNAs were also found to be differentially expressed in patients with aneurysm and to have good predictive value for diagnosing aneurysm. Said additional miRNAs are: hsa-miR-574-5p, hsa-miR-142-3p, hsa-miR-145-5p, hsa-miR-143-3p, and hsa-miR-338-5p. The complete list of miRNAs disclosed in the present application is provided in table 1.

**Table 1: Plasma miRNAs as potential biomarkers for the diagnosis of aortic and intracranial aneurysm.**

| **miRNA** | **miRBase Accession number (Release 22.1)** | **Sequence (5' → 3)** | **Precursor miRNA (pre-miRNA)** | **Genomic locus** |
|---|---|---|---|---|
| hsa-miR-199a-3p | MIMAT0000232 | ACAGUAGUCUGCACAUUGGUUA (SEQ ID NO: 1) | MIR199A1 | 19p13.2 (within Intron 15 of *DNM2* gene) |
| hsa-miR-223-3p | MIMAT0000280 | UGUCAGUUUGUCAAAUACCCCA (SEQ ID NO: 2) | MIR223 | Xq12 (within the sequence of IncRNA MIR223HG) |
| hsa-miR-335-5p | MIMAT0000765 | UCAAGAGCAAUAACGAAAAAUGU (SEQ ID NO: 3) | MIR335 | 7q32.2 (within Intron 2 of *MEST* gene) |
| hsa-miR-574-5p | MIMAT0004795 | UGAGUGUGUGUGUGUGAGUGUGU (SEQ ID NO: 4) | MIR574 | 4p14 (within Intron 1 of *FAM114A1* gene) |
| hsa-miR-142-3p | MIMAT0000434 | UGUAGUGUUUCCUACUUUAUGGA (SEQ ID NO: 5) | MIR142 | 17q22 (within the sequence of IncRNA TSPOAP1-AS1) |
| hsa-miR-145-5p | MIMAT0000437 | GUCCAGUUUUCCCAGGAAUCCCU (SEQ ID NO: 6) | MIR145 | 5q32 (within the sequence of IncRNA CARMN) |
| hsa-miR-143-3p | MIMAT0000435 | UGAGAUGAAGCACUGUAGCUC (SEQ ID NO: 7) | MIR143 | 5q32 (within the sequence of IncRNA CARMN) |
| hsa-miR-338-5p | MIMAT0004701 | AACAAUAUCCUGGUGCUGAGUG (SEQ ID NO: 8) | MIR338 | 17q25.3 (within Intron 7 of *AATK* gene) |

The methods according to the first to fifth aspects of the disclosure comprise determining in an isolated biological sample from the subject the level of at least one microRNA (miRNA) selected from the group consisting of hsa-miR-223-3p, hsa-miR-199a-3p, and hsa-miR-335-5p. In some embodiments, the methods further comprise determining the level of at least one additional miRNA selected from the group consisting of hsa-miR-574-5p, hsa-miR-142-3p, hsa-miR-145-5p, hsa-miR-143-3p, and hsa-miR-338-5p. In some embodiments the methods comprise determining at least hsa-miR-223-3p and hsa-miR-199a-3p. In other embodiments the methods comprise determining at least hsa-miR-223-3p and hsa-miR-335-5p. In other embodiments the methods comprise determining at least hsa-miR-143-3p and hsa-miR-335-5p. In particular embodiments, the methods comprise determining at least all three of hsa-miR-223-3p, hsa-miR-199a-3p and hsa-miR-335-5p. In particular embodiments, the methods comprise determining four or five miRNAs. In other particular embodiments, the methods comprise determining six miRNAs. In other particular embodiments, the methods comprise determining seven miRNAs. In particular embodiments, the methods comprise determining one of the miRNA combinations disclosed in Table 5. In more particular embodiments, the methods comprise determining all of hsa-miR-223-3p, hsa-miR-199a-3p, hsa-miR-335-5p, hsa-miR-574-5p, hsa-miR-142-3p, hsa-miR-145-5p, hsa-miR-143-3p, and hsa-miR-338-5p.

In some embodiments, when the level determined for the one or more miRNAs is at least 50% downregulated (0.5-fold change) than the corresponding reference value this is indicative that the subject suffers from an aneurysm. In particular embodiments, when the level of determined miRNA is below the threshold detailed in table 4 (see cut-off value column), this is indicative that the subject suffers from an aneurysm. In a particular embodiment, when the level determined for one or more miRNAs is 80% downregulated (0.2-fold change) than a reference value this is indicative that the subject suffers from risk of rupture of an aneurysm. In a more particular embodiment, when the level of determined miRNA is below the threshold detailed in table 5, this is indicative that the subject suffers from risk of rupture of an aneurysm.

The methods disclosed herein contemplate determining the miRNAs in any biological sample. For example, the miRNAs may be determined in tissular material, for example, in vascular tissue. However, in preferred embodiments the biological sample is a bodily fluid, such as blood, plasma, saliva, urine, cerebrospinal fluid, or semen. In particular embodiments, the biological sample is selected from peripheral blood or plasma. In this sense, it is remarkable that circulating blood miRNAs may provide an accurate diagnosis of aneurysm.

The methods disclosed herein require determining the level of miRNA. In the sense of the present disclosure the expressions "determining the level of miRNA" and "determining the level of expression of miRNA" are used interchangeably. In some embodiments, the amount of miRNA of the tested subject is quantified and compared to the corresponding reference value, which is the amount of the same miRNA of the control subject or the average amount of said miRNA of the group of control subjects. The miRNA sequences involved in the present diagnostic are disclosed in Table 1.

Determining the level of miRNA can be performed by any method known to the skilled person, provided that said method permits the detection and, at least, relative quantification of miRNA in a biological sample. Included among the examples of these procedures are polymerase chain reaction (PCR), quantitative real-time PCR (QPCR), multiplex PCR, NASBA, LCR, RT-PCR, RNA sequencing, array hybridization or "Northern" transfer, or combinations of these. In most methods of detection and quantification of RNA mentioned above, it is first necessary to convert the RNA to complementary DNA (cDNA). This conversion is accomplished by known techniques by skilled in the art, such as reverse transcription, among others.

In a particular embodiment of the methods of the disclosure the expression level of the miRNA is determined by quantification of the miRNA. In more particular embodiments this is done by reverse transcription followed by real-time quantitative PCR. For this technique, as well as for many other techniques for detecting/quantifying miRNA expression, use of amplification primers is required. In a preferred embodiment of the present invention, the primer sequences are derived from the transcript sequences of the genomic locus disclosed in Table 1. A skilled person may design appropriate primers for amplification of any nucleotide sequence by methods well known in the art. For example, the primers used for determining the level of miRNA are, for each miRNA, those shown in Table 3 (see examples below). However, other primers may be designed for use in the present methods. Determining the level of miRNA is described in further detail in the examples below.

The present methods generally require comparing the level of miRNA with a reference value. The reference value, as mentioned above, is obtained from a control subject or group of control subjects. The skilled person may use any available method to establish the described comparison. For instance, as method of relative quantification, the 2^{-ΔΔCt} of Livak and Schmittgen may be employed (Methods, 2001 vol. 25, issue 4, p.402-8).

In another embodiment, microarrays are used which include one or more probes corresponding to one or more of biomarkers (miRNAs) identified in Table 1. This method results in the production of hybridization patterns of labelled target nucleic acids on the array surface. The resultant hybridization patterns of labelled nucleic acids may be visualized or detected in a variety of ways, with the particular manner of detection selected based on the particular label of the target nucleic acid. Representative detection means include scintillation counting, autoradiography, fluorescence measurement, calorimetric measurement, light emission measurement, light scattering, and the like.

While providing for a reliable and early diagnosis of aneurysm, including information on the progression of the disease and the risk of rupture, the present diagnosis method is useful to a clinician in the sense that the method enables him/her to take the most appropriate decisions to treat the patient. Since the treatment regime may depend on the size of the aneurysm, and particularly, whether there is a risk for rupture, the clinician may, in view of the diagnosis performed as explained above, recommend the most appropriate therapy, including surgical intervention.

Recommendation for surgical intervention could also be advised even in small diameter aneurysms in case they are expressing relatively low levels of the biomarkers herein disclosed, given the fact that, although rare, there are small aneurysms prone to rupture, and there are sporadic cases of ruptured aneurysms with relatively small diameters. The biomarkers herein disclosed are overall a good indicator of prognosis regarding the risk for rupture of the aneurysm. Thus, in sporadic cases, a small diameter aneurysm may result in very low levels of expression of the disclosed biomarkers, which would be nevertheless indicative of bad prognosis and high risk for rupture, thus providing very useful information for the clinical management of the particular patient. The clinician may also recommend follow-up and close monitoring of the subject.

Accordingly, aspects three to five of the disclosure refer to a method of recommending a medical regimen for a subject suspicious of suffering from aneurysm and a method for determining the response to a medical regime for aneurysm (or similar applications of the present diagnosis).

Also contemplated is a method for diagnosing and treating aneurysm, said method comprising
i) providing a biological sample from the subject;
ii) determining the level of at least one miRNA selected from the group consisting of hsa-miR-223-3p, hsa-miR-199a-3p and hsa-miR-335-5p in the biological sample;
iii) determining that the subject suffers from aneurysm when the level determined in ii) is lower than a corresponding reference value; and
iv) administering an appropriate treatment to the subject diagnosed as having an aneurysm.

Also contemplated is a method for determining if a subject has a high risk of rupture of an aneurysm and treating the subject, said method comprising
i) providing a biological sample from the subject;
ii) determining the level of at least one miRNA selected from the group consisting of hsa-miR-223-3p, hsa-miR-199a-3p and hsa-miR-335-5p in the biological sample;
iii) determining that the subject suffers from high risk of rupture of an aneurysm when the level determined in ii) is 80% lower (0.2-fold change) than a corresponding reference value; and
iv) administering an appropriate treatment to the subject.

Therapeutic regimes for treating aneurysm include pharmacological treatment and/or nutritional intervention. Depending on the case, surgery may be required. In this sense, typical approaches are surgical replacement of the aneurysm by a synthetic graft or endovascular approach and stent grafting in an attempt to isolate the aneurysmal part of the vessel from the circulation of the blood. Usually, the regime for treating aneurysm is selected based on the anatomy and the location of the aneurysm, (there are certain anatomical restrictions that totally exclude the possibility of the endovascular approach), as well as the age and the general condition of the patient.

In some embodiments, the method is for recommending an appropriate therapeutic regime for patients at high risk for rupture of the blood vessel when the diagnosis indicates that such risk exists. Usually, the appropriate therapy in these cases is a surgical intervention, either open surgery or endovascular therapy with stent graft implantation with minimal invasive approach. In a particular embodiment, the surgery is selected from the group consisting of endovascular aneurysm repair (EVAR) and open surgery in the case of aortic aneurysms, and endovascular coiling, microvascular clipping, and catheter embolization in the case of brain aneurysms.

Pharmacological treatment may be recommendable on its own or in conjunction with surgery. In one embodiment, the therapeutic regime comprises administering a medical therapy including an anticholesterolemic, beta-blocker, anti-hypertensive, or a compound with vasodilation effect. In another embodiment, the therapeutic regime comprises gene therapy. In particular embodiments, the gene therapy comprises administering oligonucleotides, for example, small interference RNA (siRNAs) or antisense oligonucleotides (AONs). In particular embodiments, said oligonucleotides induce gene silencing or reduce the expression of one or more genes selected from the group consisting of COL5A2, COL11A1, MMP9, ITGB1, TGFB1 and BMP1. In particular embodiments, the oligonucleotides are a combination of at least six oligonucleotides, each reducing the expression of a different gene selected from the group consisting of COL5A2, COL11A1, MMP9, ITGB1, TGFB1 and BMP1. In particular embodiments, the oligonucleotides comprise a sequence that is complementary to a target sequence of a RNA transcript of COL5A2, COL11A1, MMP9, ITGB1, TGFB1 or BMP1. In particular embodiments, the oligonucleotides are 18-25 nucleotides long. In particular embodiments, the oligonucleotides are selected from small interfering RNAs (siRNAs), micro RNAs (miRNAs), and antisense oligonucleotides (AONs).

It is well-established in the clinical setting to monitor the patients with aneurysms with computer tomography scans every six months prior to surgery (until the aortic diameter reaches the point of the surgical intervention), and also, every six months postoperatively after surgical intervention during the first year and then annually. Similarly, the present diagnostic blood test can be used for a reliable monitoring of patients with aneurysm following therapeutic interventions, including also the cases of endovascular stenting interventions. In the case of the endovascular interventions, there are many cases in which the aneurysm continues to expand in diameter, despite its isolation from the circulation and the effect of the blood pressure, resulting in devastating complications such as stent migration or endoleak complications. The present blood diagnostic test also has the potential to be a good prognostic indicator for the complications of endovascular procedures. Because the blood test is more convenient and cost-effective, also without the adverse effects of the radiation when compared to the serial computer tomography scans, or the risk of renal failure because of the use of contrast, it can be used for safe and non-invasive monitoring of patients with aneurysms. The herein disclosed diagnostic blood test could be used every three months in order to detect the changes in aneurysm diameter/size earlier. It can also be used prior to the scanning of the patients with computer tomography, in such a way that, if the blood diagnostic test reveals maintained or increased levels of the miRNAs disclosed herein, this would suggest postponement of the computer tomography scanning for a future time point when its contribution to the potential diagnosis could be better justified.

The present methods are not restricted to a particular type of aneurysm. In some embodiments, the aneurysm is selected from aortic aneurysm, which can be either thoracic (ascending thoracic or arch or descending thoracic aortic aneurysm), abdominal, or thoracoabdominal, cerebral arterial aneurysm, iliac artery aneurysm, and subclavian artery aneurysm. In particular embodiments, the aneurysm is thoracic aortic aneurysm, abdominal aortic aneurysm, or intracranial aneurysm.

A sixth aspect of the disclosure refers to use of a combination of miRNAs comprising at least hsa-miR-199a-3p, hsa-miR-223-3p, and hsa-miR-335-5p as biomarker for diagnosing aneurysm or for the prognosis of an aneurysm, including risk of rupture, or for predicting relapses in a patient treated for an aneurysm. The combination of these biomarkers can diagnose, determine prognosis or relapse more accurately.

Some embodiments of the sixth aspect contemplate using one or more additional biomarkers, in particular, said one or more additional biomarkers are selected from the group consisting of hsa-miR-574-5p, hsa-miR-142-3p, hsa-miR-145-5p, hsa-miR-143-3p, and hsa-miR-338-5p. A particular embodiment provides for use of a combination of at least four, or at least five miRNAs . Another particular embodiment provides for use of a combination of at least six miRNAs. A more particular embodiment provides for use of a combination of at least seven miRNAs. One particular embodiment provides for the use of a combination of miRNAs comprising at least hsa-miR-199a-3p, hsa-miR-223-3p, hsa-miR-335-5p and hsa-miR-574-5p. Particular embodiments also provide for use of the combinations of miRNAs disclosed in Table 5. An even more particular embodiment provides for use of a combination comprising at least all of hsa-miR-223-3p, hsa-miR-199a-3p, hsa-miR-335-5p, hsa-miR-574-5p, hsa-miR-142-3p, hsa-miR-145-5p, hsa-miR-143-3p, and hsa-miR-338-5p.

The seventh aspect of the present disclosure refers to use of means for determining the level of hsa-miR-223-3p, hsa-miR-199a-3p, and hsa-miR-335-5p in any of the method as defined in the first to fifth aspects of the invention. Some embodiments of this seventh aspect contemplate using means for determining the level of one or more additional miRNAs, in particular, said one or more additional miRNAs are selected from the group consisting of hsa-miR-574-5p, hsa-miR-142-3p, hsa-miR-145-5p, hsa-miR-143-3p, and hsa-miR-338-5p. Other embodiments contemplate using means for determining the level of at least four, or at least five miRNAs in any of the method as defined in the first to fifth aspects of the invention. Other particular embodiments contemplate using means for determining the level of at least six miRNAs. Other particular embodiments contemplate using means for determining the level of at least seven miRNAs. More particular embodiments contemplate using means for determining the level of the combinations of miRNAs disclosed in Table 5. A particular embodiment provides for use of means for determining the level of at least all of hsa-miR-223-3p, hsa-miR-199a-3p, hsa-miR-335-5p, hsa-miR-574-5p, hsa-miR-142-3p, hsa-miR-145-5p, hsa-miR-143-3p, and hsa-miR-338-5p.

In some embodiments, the means comprise amplification primers designed to amplify the miRNAs as shown in Table 1. In a particular embodiment, the means are comprised in the assays referenced in Table 3. Other embodiments disclosed above for the methods of the first to fifth aspects are also applicable to this seventh aspect.

The eight aspect refers to a kit comprising means for determining the level of hsa-miR-223-3p, hsa-miR-199a-3p, and hsa-miR-335-5p and use of said kit in any of the methods as defined in aspects one to five. The kits may comprise said means and instructions for their use in said methods. All embodiments described above for the "means" are applicable to the kits. In the same way, embodiments disclosed above for the methods of the first to fifth aspects are also applicable to the kits.

The kit may comprise instructions that may include information regarding thresholds, cut-off values, reference values and interpretation of results. In some embodiments, the kits may comprise additional reagents for performing the determination of the level of the miRNAs, such as, for example, reference samples, spike samples, polymerases, nucleotides, labels, buffers, and the like. The kits may also comprise a solid support. In a particular embodiment the kit may be a microarray.

The methods of the invention provide diagnostic and prognostic information. In one embodiment, the methods of the invention further comprise the steps of (i) collecting the information, and (ii) saving the information in a data carrier.

In the sense of the invention a "data carrier" is to be understood as any means that contain meaningful information data for the diagnosis or prognosis of an aneurysm, such as paper. The carrier may also be any entity or device capable of carrying the prognosis data. For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the prognosis data are embodied in a signal that may be conveyed directly by a cable or other device or means, the carrier may be constituted by such cable or other device or means. Other carriers relate to USB devices and computer archives. Examples of suitable data carrier are paper, CDs, USB, computer archives in PCs, or sound registration with the same information.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Numbered embodiments

1. An *in vitro* method for diagnosing an aneurysm in a subject, the method comprising determining in an isolated biological sample from the subject the level of one or more microRNAs (miRNAs) selected from the group consisting of hsa-miR-223-3p, hsa-miR-199a-3p, hsa-miR-335-5p, hsa-miR-574-5p, hsa-miR-142-3p, hsa-miR-145-5p, hsa-miR-143-3p, and hsa-miR-338-5p.
2. The *in vitro* method according to embodiment 1, wherein when the level determined for each of the one or more miRNAs is lower than a corresponding reference value this is indicative that the subject suffers from an aneurysm.
3. The *in vitro* method according to embodiment 2, wherein when the level determined for each of the one or more miRNAs is at least 50% lower (0.5-fold change) than a corresponding reference value, this is indicative that the subject suffers from an aneurysm.
4. The *in vitro* method according to embodiment 2, wherein when the level determined for each of the one or more miRNAs is below the corresponding cut-off value detailed in table 4, this is indicative that the subject suffers from an aneurysm.
5. An *in vitro* method for the prognosis of an aneurysm in a subject, the method comprising determining in an isolated biological sample from the subject the level of one or more microRNAs (miRNAs) selected from the group consisting of hsa-miR-223-3p, hsa-miR-199a-3p, hsa-miR-335-5p, hsa-miR-574-5p, hsa-miR-142-3p, hsa-miR-145-5p, hsa-miR-143-3p, and hsa-miR-338-5p.
6. The *in vitro* method according to embodiment 5, wherein when the determined level is 80% lower (0.2-fold change) than a corresponding reference value this is indicative of bad prognosis, in particular, wherein bad prognosis is that the subject suffers from an aneurysm with high risk of rupture.
7. An *in vitro* method for determining the response of a patient that suffers from an aneurysm to a medical regime for aneurysm, the method comprising:
   (a) determining in an isolated biological sample from the subject the level of one or more miRNAs selected from the group consisting of hsa-miR-223-3p, hsa-miR-199a-3p and hsa-miR-335-5p, and comparing the level determined for each of the one or more miRNAs with the level of the same miRNA determined for the same patient before the start of the therapy or at an earlier phase of the therapy, wherein a decrease in the level of the miRNA with respect to start the therapy or earlier phase of the therapy is indicative of a bad response.
8. A method of recommending a medical regimen for a subject suspicious of suffering from aneurysm, the method comprising:
   (a) diagnosing if the subject suffers from aneurysm by the method as defined in any of the embodiments 1-4, and
   (b) recommending a therapeutic regime for aneurysm if the subject is diagnosed of suffering from an aneurysm or risk of rupture of an aneurysm.
9. A method of recommending a medical regimen for a subject suspicious of suffering from bad prognosis of an aneurysm, the method comprising:
   (a) diagnosing if the subject suffers from bad prognosis of an aneurysm by the *in vitro* method as defined in any one of embodiments 5-6, and
   (b) recommending a therapeutic regime for bad prognosis of an aneurysm if the subject has a bad prognosis.
10. A method for diagnosing and treating an aneurysm, said method comprising
   i) providing a biological sample from the subject;
   ii) determining the level of one or more microRNAs (miRNAs) selected from the group consisting of hsa-miR-223-3p, hsa-miR-199a-3p, hsa-miR-335-5p, hsa-miR-574-5p, hsa-miR-142-3p, hsa-miR-145-5p, hsa-miR-143-3p, and hsa-miR-338-5p in the biological sample;
   iii) determining that the subject suffers from aneurysm when the level of each of the one or more miRNAs determined in ii) is lower than a corresponding reference value; and
   iv) administering an appropriate treatment to the subject diagnosed as having an aneurysm.
11. A method for determining if a subject has a bad prognosis for an aneurysm and treating the subject, said method comprising
   i) providing a biological sample from the subject;
   ii) determining the level of one or more microRNAs (miRNAs) selected from the group consisting of hsa-miR-223-3p, hsa-miR-199a-3p, hsa-miR-335-5p, hsa-miR-574-5p, hsa-miR-142-3p, hsa-miR-145-5p, hsa-miR-143-3p, and hsa-miR-338-5p in the biological sample;
   iii) determining that the subject has a bad prognosis when the level of each of the one or more miRNAs determined in ii) is lower than a corresponding reference value; and
   iv) administering an appropriate treatment to the subject
12. The method according to any one of embodiments 2-4, 6-11, wherein the reference value is a predefined level of the miRNA determined from a healthy subject or a group of subjects.
13. The method according to any one of embodiments 8-12, wherein the therapeutic regime or treatment comprises administering an anticholesterolemic agent, a beta-blocker, an anti-hypertensive, or a vasodilator compound.
14. The method according to any one of embodiments 8-12, wherein the therapeutic regime or treatment comprises gene therapy.
15. The method according to the preceding embodiment, wherein the gene therapy comprises administering small interference RNA (siRNAs) or antisense oligonucleotides (AONs).
16. The method according to the preceding embodiment, wherein the siRNAs or AONs induce gene silencing of one or more gene selected from the group consisting of COL5A2, COL11A1, MMP9, ITGB1, TGFB1, and BMP1.
17. The method according to any one of embodiments 8-12, wherein the therapeutic regime or treatment comprises surgery.
18. The method according to the preceding embodiment, wherein the surgery is selected from the group consisting of Endovascular aneurysm repair (EVAR), open surgery, endovascular coiling, microvascular clipping, and catheter embolization.
19. The method according to any one of the preceding embodiments, the method comprising determining hsa-miR-223-3p.
20. The method according to any one of the preceding embodiments, the method comprising determining hsa-miR-199a-3p.
21. The method according to any one of the preceding embodiments, the method comprising determining hsa-miR-335-5p.
22. The method according to any one of the preceding embodiments, wherein the method comprises determining the level of hsa-miR-223-3p, hsa-miR-199a-3p and hsa-miR-335-5p.
23. The method according to any one of the preceding embodiments, further comprising determining the level of hsa-miR-574-5p.
24. The method according to any one of the preceding embodiments, further comprising determining the level of hsa-miR-142-3p.
25. The method according to any one of the preceding embodiments, further comprising determining the level of hsa-miR-145-5p.
26. The method according to any one of the preceding embodiments, further comprising determining the level of hsa-miR-143-3p.
27. The method according to any one of the preceding embodiments, further comprising determining the level of hsa-miR-338-5p.
28. The method according to any one of the preceding embodiments, said method comprising determining the level of a combination of miRNAs as disclosed in Table 5.
29. The method according to the preceding embodiment, wherein the method comprises determining the level of hsa-miR-223-3p, hsa-miR-199a-3p, hsa-miR-335-5p, hsa-miR-574-5p, hsa-miR-142-3p, hsa-miR-145-5p, hsa-miR-143-3p, and hsa-miR-338-5p.
30. The method according to any one of the preceding embodiments, wherein the biological sample is a bodily fluid.
31. The method according to the preceding embodiment, wherein the biological sample is selected from blood, plasma, saliva, urine, cerebrospinal fluid, and semen.
32. The method according to the preceding embodiment, wherein the biological sample is selected from peripheral blood or plasma.
33. The method according to the preceding embodiment, wherein the biological sample is tissue material.
34. The method according to the preceding embodiment, wherein the biological sample is vascular tissue.
35. The method according to any one of the preceding embodiments, wherein the level of miRNA is determined by Reverse Transcription Quantitative Polymerase Chain Reaction (RT-qPCR).
36. Use of one or more microRNAs (miRNAs) selected from the group consisting of hsa-miR-223-3p, hsa-miR-199a-3p, hsa-miR-335-5p, hsa-miR-574-5p, hsa-miR-142-3p, hsa-miR-145-5p, hsa-miR-143-3p, and hsa-miR-338-5p as an *in vitro* biomarker for the diagnosis or prognosis of aneurysm, including risk of rupture of an aneurysm.
37. The use according to the preceding embodiment, wherein the biomarker hsa-miR-223-3p is selected.
38. The use according to embodiment 36, wherein the biomarker hsa-miR-199a-3p is selected.
39. The use according to embodiment 36, wherein the biomarker hsa-miR-335-5p is selected.
40. The use according to embodiment 36, wherein the biomarkers hsa-miR-223-3p, hsa-miR-199a-3p and hsa-miR-335-5p are selected.
41. The use according to any one of embodiments 36-40, wherein the biomarker hsa-miR-574-5p is additionally selected.
42. The use according to any one of embodiments 36-41, wherein the biomarker hsa-miR-142-3p is additionally selected.
43. The use according to any one of embodiments 36-42, wherein the biomarker hsa-miR-145-5p is additionally selected.
44. The use according to any one of embodiments 36-43, wherein the biomarker hsa-miR-143-3p is additionally selected.
45. The use according to any one of embodiments 36-44, wherein the biomarker hsa-miR-338-5p is additionally selected.
46. The use according to embodiment 36, wherein a combination of miRNAs as disclosed in Table 5 is selected.
47. Use of means for determining the level of one or more microRNAs (miRNAs) selected from the group consisting of hsa-miR-223-3p, hsa-miR-199a-3p, hsa-miR-335-5p, hsa-miR-574-5p, hsa-miR-142-3p, hsa-miR-145-5p, hsa-miR-143-3p, and hsa-miR-338-5p, in a method as defined in any of the embodiments 1-35.
48. Use of means according to the preceding embodiment, wherein the means comprise primers for performing RT-qPCR.
49. Use according to the preceding embodiment, wherein the means are comprised in the assays referenced in table 3.
50. A kit comprising means for determining the level of one or more microRNAs (miRNAs) selected from the group consisting of hsa-miR-223-3p, hsa-miR-199a-3p, hsa-miR-335-5p, hsa-miR-574-5p, hsa-miR-142-3p, hsa-miR-145-5p, hsa-miR-143-3p, and hsa-miR-338-5p.
51. The kit according to the preceding embodiment, wherein the kit comprises means for determining hsa-miR-223-3p.
52. The kit according to embodiment 50, wherein the kit comprises means for determining hsa-miR-199a-3p.
53. The kit according to embodiment 50, wherein the kit comprises means for determining hsa-miR-335-5p.
54. The kit according to any one of embodiments 50-53, wherein the kit comprises means for determining hsa-miR-223-3p, hsa-miR-199a-3p, and hsa-miR-335-5p.
55. The kit according to any one of embodiments 50-54, wherein the kit comprises means for determining hsa-miR-574-5p.
56. The kit according to any one of embodiments 50-55, wherein the kit comprises means for determining hsa-miR-142-3p.
57. The kit according to any one of embodiments 50-56, wherein the kit comprises means for determining hsa-miR-145-5p.
58. The kit according to any one of embodiments 50-57, wherein the kit comprises means for determining hsa-miR-143-3p.
59. The kit according to any one of embodiments 50-58, wherein the kit comprises means for determining has-miR-338-5p.
60. The kit according to any one of embodiments 50-59, wherein the means comprise primers for performing RT-qPCR.
61. The kit according to any one of embodiments 50-60, wherein the kit comprises means for determining a combination of miRNAs of Table 5.
62. The kit according to the preceding embodiment, wherein the means are comprised in the assays referenced in table 3.
63. Use of a kit as defined in the preceding embodiment in a method as defined in any one of embodiments 1-35.
64. The method according to any one of embodiments 1-35, or the use according to any one of embodiments 36-46, wherein the aneurysm is aortic aneurysm.
65. The method according to any one of embodiments 1-35, or the use according to any one of embodiments 36-46, wherein the aneurysm is abdominal aortic aneurysm or thoracic aortic aneurysm.
66. The method according to any one of embodiments 1-35, or the use according to any one of embodiments 36-46, wherein the aneurysm is intracranial aneurysm.

### Examples

### 1. Materials and methods

### 1.1 Clinical subjects

A total of 80 patients with aneurysm and 51 healthy adults were recruited for this study (Table 2). Among the aneurysm patients, 22 were diagnosed with thoracic aortic aneurysm, 38 with abdominal aortic aneurysm and 20 patients with intracranial aneurysm. The two groups were comparable with respect to age and gender (Table 2). Written informed consent was obtained from all subjects involved in the study.

**Table 2: General characteristics of the clinical groups of the study.**

| Clinical group | Number of subjects | Gender (Male/Female) | Age (years) |
|---|---|---|---|
| Aneurysm patients | 80 | 65/15 | 71.7 |
| Controls | 51 | 35/16 | 70.1 |
| Total | 131 | 100/31 | 70.9 |

The group of patients with aneurysm was further divided into the subgroups of small (n=47) and large aneurysms (n=33) according to their size (maximal diameter). Specifically, aortic aneurysms with diameter >5.5 cm and intracranial aneurysms with diameter >15 mm were classified as large aneurysms.

### 1.2 Blood plasma collection and processing

Up to 10 mL peripheral venous blood from each participant was collected into K₂EDTA tubes, placed on wet ice in upright position and proceeded to centrifuge at 1,000 × g for 15 min at 4°C within 120 minutes after blood collection. The upper plasma phase was transferred to a new tube, without disturbing the intermediate buffy coat layer and centrifuged at 3,000 × g for 15 min at 4°C to obtain platelet poor plasma. Plasma samples were fractioned into multiple aliquots and stored at - 80 °C until further analysis.

### 1.3 Plasma RNA isolation

Total RNA was extracted from plasma samples using the miRNeasy Mini Kit (Qiagen, Germany). For each sample, 200 µL of plasma was homogenized with 1000 µL of the Qiazol reagent. A synthetic RNA oligonucleotide mix obtained from the miRCURY Spike-In kit (Qiagen, Germany) was added to each sample at equimolar amounts prior to RNA extraction. These spike-ins will be subsequently used to monitor RNA extraction efficiency. After incubation at room temperature for 15 minutes, 200 µL of chloroform was added to the lysates followed by centrifugation at 12,000 × g for 15 minutes at 4°C. Precisely 650 µL of the upper aqueous phase was mixed with 7 µL glycogen (Ambion, 5mg/mL) to enhance precipitation. Samples were transferred to a miRNeasy mini column and RNA was precipitated with ethanol followed by washing with RPE and RWT buffers. Finally, total RNA was eluted in 30 µL nuclease free water and stored at -80°C until further analysis.

### 1.4 Reverse transcription and qPCR

Starting from total RNA samples, cDNA was synthesized using the miRCURY RT Kit (Qiagen, Germany). Reaction conditions were set according to recommendations by the manufacturer and 2 µL of total RNA was used as an input in a 10 µL reaction. Quantitative PCR reactions were performed using the miRCURY SYBR^{®} Green master mix (Qiagen, Germany) and miRCURY LNA-enhanced primer assays (Qiagen, Germany) (Table 3) on the LightCycler 480 II instrument (Roche, Germany). cDNA samples were diluted 1:100 and the temperature settings were as follows: 95°C for 10 min, 45 cycles of 95°C for 10 s and 56°C for 60 s, followed by melting curve analysis. The second derivative maximum method was used to calculate the cycle of quantification values (Cq-values). miRNA expression data were normalized to UniSp4, a synthetic RNA spike-in control.

**Table 3: miRCURY LNA miRNA PCR assays by Qiagen used for the quantification of miRNA-targets.**

| miRNA | miRCURY LNA miRNA PCR assay (GeneGlobe ID) by Qiagen |
|---|---|
| hsa-miR-199a-3p | YP00204536 |
| hsa-miR-223-3p | YP00205986 |
| hsa-miR-335-5p | YP02119293 |
| hsa-miR-574-5p | YP02116206 |
| hsa-miR-142-3p | YP00204291 |
| hsa-miR-145-5p | YP00204483 |
| hsa-miR-143-3p | YP00205992 |
| hsa-miR-338-5p | YP00204114 |
| UniSp4 | YP00203953 |

### 1.5 Statistical analysis

Statistical analysis was performed using the GraphPad Prism 9.5.0 software. To compare the differences in expression levels of plasma miRNAs between the two groups, we conducted the Mann-Whitney U test. Receiver operating characteristic (ROC) curves were constructed for each miRNA and the area under ROC curve (AUC) was determined, as well. Sensitivity and specificity in discriminating aneurysm patients were also estimated for each miRNA. Multivariate logistic regression analysis was performed to determine the combined prognostic probability of miRNAs.

### 2. RESULTS

As shown in Figure 1, the expression levels of the eight miRNAs were significantly downregulated in the group of patients with aneurysm compared to the controls. In addition, ROC curve analysis was conducted for each miRNA individually to further evaluate the predictive value of differentially expressed miRNAs in distinguishing aneurysm patients from controls. The area under ROC curve (AUC) values ranged from 0.78 - 0.98, demonstrating that all miRNAs have strong predictive value as diagnostic biomarkers of aneurysm.

In the group of aneurysm it was additionally found that miRNA levels in plasma were equivalent in aortic (thoracic and abdominal) and intracranial aneurysms, resulting in the clustering of all aneurysm subjects.

In Table 4 the fold change in the expression of each miRNA in the aneurysm group was determined relative to the control group. The sensitivity and specificity and the suggested cut-off values were estimated based on the ROC curve. For six of the miRNAs, sensitivity was calculated >81% while the corresponding specificity was >76%. The highest values of sensitivity and specificity were reported for hsa-miR-223-3p, 92.5% and 86.27%, respectively, followed by hsa-miR-199a-3p and hsa-miR-335-5p.

The combined prognostic probability of two, three, four, five, six, seven and all of the diagnostic miRNAs was estimated in a single model, using multivariate logistic regression analysis (Table 5). The combination of 6 miRNA biomarkers improved the predictive value to 97.5% sensitivity and 90.2% specificity, while the combination of 7 or all of the miRNAs generated the best predictive value of 100% sensitivity and 92.16% specificity.

Table 4: Results of statistical analysis between the groups of aneurysm and control using Kruskal-Wallis test. The fold change of plasma miRNA expression in the group of aneurysm is determined relative to expression in healthy controls. The ROC curve analysis parameters of sensitivity, specificity, cut-off values and AUC are also estimated.

| **miRNA** | **Fold change** | **P value** | **Sensitivity (%)** | **Specificity (%)** | **AUC (95% CI)** | **Cut-off value** |
|---|---|---|---|---|---|---|
| hsa-miR-199a-3p | 0.170 | <0.0001 | 91.25 | 84.31 | 0.9379 (0.90-0.98) | <-4.025 |
| hsa-miR-223-3p | 0.213 | <0.0001 | 92.50 | 86.27 | 0.963 (0.93-0.99) | <0.87 |
| hsa-miR-335-5p | 0.233 | <0.0001 | 90 | 86.27 | 0.964 (0.94-0.99) | <-7.715 |
| hsa-miR-574-5p | 0.322 | <0.0001 | 81.25 | 76.47 | 0.902 (0.85-0.95) | <-9.95 |
| hsa-miR-142-3p | 0.320 | <0.0001 | 81.25 | 84.31 | 0.891 (0.82-0.96) | <-1.3 |
| hsa-miR-145-5p | 0.441 | <0.0001 | 83.75 | 82.35 | 0.859 (0.79-0.92) | <-5.395 |
| hsa-miR-143-3p | 0.505 | <0.0001 | 66.25 | 68.63 | 0.759 (0.68-0.84) | <-5.21 |
| hsa-miR-338-5p | 0.446 | <0.0001 | 72.15 | 68.63 | 0.737 (0.65-0.82) | <-11.18 |

**Table 5: miRNA combinations with the strongest predictive value to discriminate patients with aneurysm using logistic regression models. ROC curves for each miRNA signature are also depicted.**

| | **Sensitivity (%)** | **Specificity (%)** |
|---|---|---|
| miR-199a-3p & miR-223-3p | 93.75 | 86.27 |
| miR-199a-3p & miR-223-3p & miR-335-5p | 91.25 | 84.31 |
| miR-223-3p & miR-335-5p | 92.5 | 86.27 |
| miR-199a-3p & miR-223-3p & miR-335-5p & miR-574-5p | 93.75 | 84.31 |
| miR-223-3p & miR-335-5p & miR-574-5p | 92.5 | 86.27 |
| miR-199a-3p & miR-223-3p & miR-335-5p & miR-574-5p & miR-142-3p | 93.75 | 86.27 |
| miR-199a-3p & miR-223-3p & miR-335-5p & miR-574-5p & miR-142-3p & miR-145-5p | 97.5 | 90.2 |
| miR-199a-3p & miR-223-3p & miR-335-5p & miR-574-5p & miR-142-3p & miR-145-5p & miR-143-3p | 100 | 92.16 |
| miR-199a-3p & miR-223-3p & miR-335-5p & miR-574-5p & miR-142-3p & miR-145-5p & miR-143-3p & miR-338-5p | 100 | 92.16 |

Furthermore, the expression levels of the 4 most significant miRNAs, i.e. hsa-miR-199a-3p, hsa-miR-223-3p, hsa-miR-335-5p and hsa-miR-574-5p were analyzed in the subgroups of aneurysm patients (small diameter versus large diameter aneurysms). Statistically significant differences in the expression of each miRNA were observed (p<0.0001) between the two subgroups. Decreased levels of expression were determined in subjects with large diameter aneurysms in comparison to patients with small diameter aneurysms. Patients with large aneurysms showed greater differences in miRNAs expression compared to controls; fold change determination indicated downregulation of 89% (0.11-fold change), 85% (0.15-fold change), 83% (0.17-fold change) and 79% (0.21-fold change) in miR-199a-3p, miR-223-3p, miR-335-5p and miR-574-5p expression, respectively, in the group of large aneurysms (Table 6). Smaller differences in miRNAs expression were observed in patients with small aneurysms; miR-199a-3p expression was 75% downregulated (0.25-fold change), miR-223-3p & miR-335-5p levels were 71% downregulated (0.29-fold change) and miR-574-5p was 53% downregulated (0.47-fold change) as shown in Table 6 (Controls, representing healthy subjects, were set as the reference group for these comparisons).

**Table 6: Fold change in miRNAs expression in the groups of small and large aneurysms compared to controls.**

| **miRNA** | **Mean Normalized Ct value of Small Aneurysms (N = 47)** | **Mean Normalized Ct value of Large Aneurysms (N = 33)** | **Mean Normalized Ct value of Controls (N = 51)** | **Fold change in Small Aneurysms** | **Fold change in Large Aneurysms** |
|---|---|---|---|---|---|
| hsa-miR-199a-3p | -4.95 | -6.13 | -2.94 | 0.25 | 0.11 |
| hsa-miR-223-3p | 0.12 | -0.79 | 1.91 | 0.29 | 0.15 |
| hsa-miR-335-5p | -8.56 | -9.30 | -6.77 | 0.29 | 0.17 |
| hsa-miR-574-5p | -10.46 | -11.63 | -9.36 | 0.47 | 0.21 |

The prognostic probability of each miRNA individually and of combinations of miRNAs in discriminating patients with small versus large aneurysms was evaluated by ROC curve analysis. Hsa-miR-199a-3p was shown to be the best single prognostic biomarker for large aneurysms with sensitivity 87.88% and specificity 89.36% (Table 7 & Figure 2A).

**Table 7: Estimation of the predictive value of miRNA biomarkers in distinguishing patients with large and small aneurysms. The ROC curve analysis parameters of sensitivity, specificity, cut-off values and AUC are also given.**

| **miRNA** | **P value** | **Sensitivity (%)** | **Specificity (%)** | **AUC (95% CI)** | **Cut-off value** |
|---|---|---|---|---|---|
| hsa-miR-199a-3p | <0.0001 | 87.88 | 89.36 | 0.94 (0.90-0.99) | <-5.645 |
| hsa-miR-223-3p | <0.0001 | 87.88 | 80.85 | 0.88 (0.80-0.96) | <-0.46 |
| hsa-miR-335-5p | <0.0001 | 69.70 | 68.09 | 0.75 (0.64-0.86) | <-8.95 |
| hsa-miR-574-5p | <0.0001 | 75.76 | 72.34 | 0.83 (0.73-0.92) | <-10.86 |

The combination of hsa-miR-199a-3p and hsa-miR-223-3p through multivariate logistic regression analysis, improved the prognostic probability to 93.94% sensitivity and 95.74% specificity (Figure 2E). The combination of all 4 miRNAs increased further the prognostic probability in discriminating large diameter aneurysms from small diameter aneurysms reaching 96.89% sensitivity and 97.55% specificity (Figure 2F).

### Citation List

EP3794148
Methods, 2001 vol. 25, issue 4, p.402-8

## Claims

1. An *in vitro* method for diagnosing an aneurysm in a subject, the method comprising determining in an isolated biological sample from the subject the level of one or more microRNAs (miRNA) selected from the group consisting of hsa-miR-223-3p, hsa-miR-199a-3p and hsa-miR-335-5p, wherein, when the level of the one or more miRNAs is lower than a corresponding reference value, this is indicative that the subject suffers from an aneurysm.

2. An *in vitro* method for determining the risk of rupture of an aneurysm in a subject, the method comprising determining in an isolated biological sample from the subject the level of one or more microRNA (miRNA) selected from the group consisting of hsa-miR-223-3p, hsa-miR-199a-3p, hsa-miR-335-5p and hsa-miR-574-5p, wherein, when the level of the one or more MiRNAs is 80% lower (0.2-fold change) than a corresponding reference value this is indicative that the subject suffers from risk of rupture of an aneurysm.

3. The *in vitro* method according to any one of claims 1-2, wherein the method comprises determining the level of at least hsa-miR-223-3p, hsa-miR-199a-3p and hsa-miR-335-5p, wherein, when the level of each one of hsa-miR-223-3p, hsa-miR-199a-3p, hsa-miR-335-5p and hsa-miR-574-5p is lower than a corresponding reference value, this is indicative that the subject suffers from an aneurysm.

4. The *in vitro* method according to claim 3, further comprising determining the level of at least one additional miRNA selected from the group consisting of hsa-miR-574-5p, hsa-miR-142-3p, hsa-miR-145-5p, hsa-miR-143-3p, and hsa-miR-338-5p.

5. The *in vitro* method according to claim 4, wherein the method comprises determining the level of at least hsa-miR-223-3p, hsa-miR-199a-3p, hsa-miR-335-5p, hsa-miR-574-5p, hsa-miR-142-3p, hsa-miR-145-5p, hsa-miR-143-3p, and hsa-miR-338-5p, wherein, when the level of each one of hsa-miR-574-5p, hsa-miR-142-3p, hsa-miR-145-5p, hsa-miR-143-3p, and hsa-miR-338-5p is lower than a corresponding reference value, this is indicative that the subject suffers from an aneurysm.

6. The *in vitro* method according to any one of claims 1-5, wherein the corresponding reference value is a predefined level of the microRNAs determined from a healthy subject or a group of healthy subjects.

7. A method of recommending a medical regimen for a subject suspicious of suffering from aneurysm or from risk of aneurysm, the method comprising:
(a) diagnosing if the subject suffers from aneurysm by the method as defined in any of the claims 1, or 3-6, or determining if the subject suffers from risk of rupture of an aneurysm by the method as defined in any of the claims 2-6, and
(b) recommending a therapeutic regime for aneurysm if the subject is diagnosed of suffering from an aneurysm or a therapeutic regime for risk of rupture of an aneurysm if the subject is determined of suffering from risk of rupture of an aneurysm.

8. An *in vitro* method for determining the response of a patient that suffers from an aneurysm to medical regime for aneurysm, the method comprising:
(a) determining in an isolated biological sample from the subject the level of one or more miRNAs selected from the group consisting of hsa-miR-223-3p, hsa-miR-199a-3p and hsa-miR-335-5p, and comparing said level with the level of the same miRNA determined for the same patient before the start of the therapy or at an earlier phase of the therapy, wherein a decrease in the level of the miRNA with respect to start the therapy or earlier phase of the therapy is indicative of a bad response.

9. The *in vitro* method according to the preceding claim, wherein at least the level of hsa-miR-223-3p, hsa-miR-199a-3p and hsa-miR-335-5p is determined, and optionally the level of one or more additional miRNAs selected from the group consisting of hsa-miR-574-5p, hsa-miR-142-3p, hsa-miR-145-5p, hsa-miR-143-3p, and hsa-miR-338-5p, is also determined.

10. The *in vitro* method according to any one of claims 1-9, wherein the biological sample is selected from peripheral blood or plasma.

11. Use of a combination of miRNAs comprising at least hsa-miR-223-3p, hsa-miR-199a-3p and hsa-miR-335-5p, optionally further comprising one or more miRNAs selected from the group consisting of hsa-miR-574-5p, hsa-miR-142-3p, hsa-miR-145-5p, hsa-miR-143-3p, and hsa-miR-338-5p, as biomarker for diagnosing aneurysm or determining risk of rupture of an aneurysm.

12. A kit comprising means for determining the level of each one of hsa-miR-223-3p, hsa-miR-199a-3p, and hsa-miR-335-5p, and, optionally, means for determining the level of one or more miRNAs selected from the group consisting of hsa-miR-574-5p, hsa-miR-142-3p, hsa-miR-145-5p, hsa-miR-143-3p, and hsa-miR-338-5p.

13. Use of a kit as defined in claim 12 for diagnosing aneurysm by the method as defined in any of one of claims 1, 3-6, or 10, or for determining risk of rupture of an aneurysm by the method as defined in any one of claims 2-6 or 10, or for recommending a medical regimen for a subject suspicious of suffering from aneurysm or from risk of aneurysm as defined in any one of claims 7 or 10, or for determining the response of a patient that suffers from an aneurysm to medical regime for aneurysm as defined in any one of claims 8-9 or 10.

14. The method according to any one of claims 1-10, or the use of a combination of miRNAs according to claim 11, or the kit according to claim 12, or the use of a kit according to claim 13, wherein the aneurysm is selected from the group consisting of aortic aneurysm and intracranial aneurysm.
